# FASCICULE DE BREVET EUROPEEN

(11) **EP 1 302 216 B1**
(45) Date de publication et mention de la délivrance du brevet: **21.06.2006**
(21) Numéro de dépôt: 02292502.8
(22) Date de dépôt: 11.10.2002
(51) Int. Cl.: A61N 1/368, A61N 1/39

(54) **Dispositif médical actif de type défibrillateur/cardioverteur implantable comportant des moyens de détection de tachycardies sinusales post-thérapie**
Aktive medizinische Vorrichtung des Typs implantierbarer Defibrillator/Kardioverter mit Mitteln zur Detektion von Nachtherapie-Sinus-Tachykardien
Active implantable medical device of the implantable defibrillator/cardioverter type having means for detecting post-therapy sinus tachycardia

(30) Priorité: 12.10.2001 FR 0113135
(43) Date de publication de la demande: 16.04.2003
(73) Titulaire: ELA MEDICAL, 92541 Montrouge (FR)
(72) Inventeur: Henry, Christine, 75014 Paris (FR); Kroiss, Daniel, 38330 Saint Ismier (FR)
(74) Mandataire: Dupuis-Latour, Dominique

(56) Documents cités:
- EP-A- 0 626 182
- EP-A- 0 838 235
- US-A- 5 191 884
- US-A- 6 151 524
- US-B1- 6 178 350

## Description

La présente invention concerne les dispositifs médicaux implantables actifs (au sens de la directive 90/385/CEE du 20 juin 1990 du Conseil des communautés européennes), et plus particulièrement la famille des appareils chargés de délivrer au coeur des impulsions électriques de haute énergie (c'est-à-dire dépassant notablement l'énergie fournie pour la simple stimulation) en vue de mettre fin à une tachyarythmie. Ces modes de thérapie incluent également un mode de stimulation programmée à haute fréquence ou "ATP" (*AntiTachycardia Pacing*).

Ces dispositifs sont communément appelés "défibrillateurs implantables" ou "appareils de cardioversion", étant entendu que l'invention couvre aussi bien les défibrillateurs/cardioverteurs/stimulateurs implantables que les défibrillateurs/stimulateurs implantables (par "thérapie", on entendra aussi bien l'application d'un choc de défibrillation ou de cardioversion que celle d'une stimulation ATP).

Les tachycardies, dont le symptôme est un rythme cardiaque rapide, comprennent :
- les tachyarythmies (pathologiques), incluant la fibrillation ventriculaire (FV), la tachycardie ventriculaire (TV) et la tachycardie supra-ventriculaire (TSV, recouvrant elle-même la tachycardie auriculaire, le *flutter* auriculaire et la fibrillation auriculaire (FA)) ; et
- la tachycardie sinusale (TS, physiologique).

Le diagnostic et la classification des tachycardies est opéré, de manière en elle-même connue (voir notamment les EP-A-0 626 182 et EP-A-0 838 235 au nom de ELA Médical, ou encore le US-A- 6 151 524 (CPI)), à partir de critères tels que la fréquence ventriculaire, la stabilité des intervalles ventriculaires (intervalles RR), l'analyse de l'association auriculo-ventriculaire (révélée par la stabilité de l'intervalle PR) et le mode de démarrage des tachycardies (présence d'une accélération brusque et cavité d'origine, ventriculaire ou auriculaire).

Les algorithmes décrits dans les brevets précités se révèlent particulièrement efficaces pour discriminer les situations où il y a lieu de délivrer une thérapie ventriculaire (TV véritable) de celles où il n'y a pas lieu de le faire (TS ou TSV, car dans ce dernier cas la tachycardie est d'origine auriculaire et une thérapie auriculaire peut être envisagée).

La mise en oeuvre clinique de ces algorithmes de diagnostic et de décision d'application d'une thérapie a toutefois révélé une difficulté dans la période suivant immédiatement l'application d'une thérapie.

En effet, après application d'un choc ou d'une stimulation ATP, l'algorithme est réitéré pour déterminer si la thérapie doit être à nouveau appliquée ou si, au contraire, elle a permis de faire disparaître la tachyarythmie à l'origine du déclenchement de cette thérapie.

L'étude clinique a en effet révélé que certains cas de TS post-thérapie ont été interprétés à tort par l'algorithme comme des TV, et ont donc provoqué inutilement la répétition de la thérapie.

Or il est souhaitable, pour plusieurs raisons, d'éviter d'appliquer une thérapie lorsque celle-ci n'est pas nécessaire :
- l'application injustifiée d'une thérapie peut avoir des conséquences délétères sur l'évolution du rythme cardiaque du patient,
- la thérapie, notamment sous forme d'un choc de cardioversion ou de défibrillation, est douloureuse pour le patient et doit donc être limitée aux cas de nécessité absolue.
- une thérapie répétée va inquiéter le patient et le praticien, qui croiront à tort que la thérapie initiale n'a pas été efficace alors qu'en fait la répétition de la thérapie résulte seulement d'une mauvaise interprétation de la situation par l'algorithme

Le but de l'invention est de remédier à l'inconvénient précité, en proposant de perfectionner les algorithmes mis en oeuvre par les appareils existants afin d'éliminer les risques de faux diagnostics de TV correspondant à une TS post-thérapie non redevable d'une thérapie. Le dispositif médical de l'invention est un défibnllateur ou cardioverteur de type en lui-même connu tel que décrit par exemple par le

US-A-6151 524 précité, qui correspond au préambule de la revendication 1. L'invention propose le perfectionnement énoncé par la partie caractérisante de cette revendication 1.

Les sous-revendications visent diverses mises en oeuvre preférentielles de l'invention.

On va maintenant décrire un exemple de mise en oeuvre de l'invention, en référence aux dessins annexés.
La figure 1 est un organigramme montrant les différentes étapes du procédé mis en oeuvre par l'invention, pour opérer la discrimination et la classification des tachyarythmies et la commande sélective d'une thérapie.
La figure 2 est un chronogramme décrivant une séquence d'événements auriculaires et ventriculaires avant, pendant et après application d'une thérapie, conformément aux enseignements de la présente invention.

L'invention est un perfectionnement à l'algorithme déjà connu et décrit dans les EP-A-0 626 182 et EP-A-0 838 235 précités, et qui sont mis en oeuvre dans les modèles de défibrillateurs DEFENDER et ALTO d'Ela Médical pour opérer la détection et la classification des différentes tachyarythmies en fonction de divers critères (on pourra se référer à ces documents pour de plus amples détails).

Plus précisément, les critères mis en oeuvre sont :
1° la fréquence ventriculaire ;
2° la stabilité des intervalles ventriculaires (intervalles RR) : on définit qu'il y a "stabilité des intervalles RR" lorsque le pic d'autocorrélation, divisé par le total d'autocorrélation, excède un rapport déterminé (le pic d'autocorrélation est le nombre maximal d'intervalles récents dans le ventricule qui satisfont à un critère de stabilité prédéterminé) ;
3° la stabilité de conduction (stabilité de l'intervalle PR, révélée par l'analyse de l'association auriculo-ventriculaire : on définit qu'il y a stabilité de conduction lorsque la valeur du pic d'intercorrélation, divisée par la valeur du pic d'autocorrélation, excède un rapport déterminé (le pic d'intercorrélation est le nombre maximal d'intervalles de conduction en provenance de l'oreillette qui satisfont un critère de stabilité prédéterminé) ; en d'autres termes, on compare la stabilité de la conduction entre les deux chambres à celles des intervalles dans le ventricule ;
4° le type d'association auriculo-ventriculaire, en 1:1 ou n:1, en comparant le pic d'intercorrélation au total d'intercorrélation ;
5° le mode de démarrage des tachycardies : présence d'une accélération brusque prenant naissance avec une dissociation, et cavité d'origine (ventriculaire ou auriculaire) ; le critère d'analyse de l'accélération du rythme ventriculaire et la détermination de l'origine de cette accélération sont décrits dans le EP-A-0 626 182 précité ; et
6° la présence ou l'absence de cycles longs : on définit qu'il y a présence de cycles longs si l'on détecte au moins un cycle dont la durée de l'intervalle RR est supérieure à une valeur prédéfinie, par exemple exprimée sous la forme (*RRmax* + *StabRR*), *RRmax* étant la limite supérieure du pic d'autocorrélation et *StabRR* étant une valeur paramétrée définissant un intervalle de garde entre cette limite supérieure et la plage de détection des intervalles considérés comme longs.

La mise en oeuvre de cet algorithme connu permet de discriminer entre :
- d'une part, une tachycardie ventriculaire (TV), qui est une tachyarythmie qui a probablement son origine dans le ventricule, et qui est susceptible d'être interrompue par une thérapie appliquée à cette chambre ; une action sur le ventricule doit donc être autorisée ;
- d'autre part, une tachycardie supra-ventriculaires (TSV) ou une tachycardie sinusale (TS, vraisemblablement d'origine physiologique), qui ne sont en aucune façon susceptibles d'être interrompues par une action sur le ventricule, parce qu'elles ont leur origine dans l'oreillette ; toute thérapie ventriculaire doit donc être inhibée (une action sur l'oreillette pouvant être éventuellement déclenchée dans le cas d'une TSV).

La mise en oeuvre de cet algorithme connu permet, en tant que de besoin, de délivrer une thérapie lorsqu'une TV véritable est détectée, en éliminant les cas de faux diagnostic de TV correspondant à une TSV.
Jusqu'à présent, dans les dispositifs de l'art antérieur, après qu'une thérapie ait été appliquée, cet algorithme était réitéré, tel quel, afin de traiter à nouveau, si nécessaire, une tachyarythmie récidivante.
Cela étant, on a pu observer dans certains cas cliniques des faux diagnostics, certaines TS post-thérapie étant interprétées (à tort) comme des TV.

Ces faux diagnostics correspondent en fait à une situation où l'on a : intervalles RR stables / association / association en 1:1 / accélération brusque du ventricule (concernant ce dernier critère, dans la mesure où, juste après une thérapie, on ne dispose pas encore de données sur l'accélération en début d'arythmie, l'algorithme force, par défaut, le critère d'accélération à "accélération brusque du ventricule").

Ce faux diagnostic déclenche l'application à nouveau d'une thérapie, alors que celle-ci n'était pas nécessaire car, s'agissant d'une TS, l'application d'un choc sera sans effet, cette TS pouvant au surplus être d'origine simplement physiologique.

Le nombre d'observations d'un tel cas est en outre accentué par la programmation d'un intervalle de détection long.

Il peut s'agir également de TV lentes (fréquence cardiaque dans la plage 100-150 cpm) qui peuvent être supportées un certain temps par le patient sans qu'il soit nécessaire d'appliquer un choc, à la différence des TV rapides (fréquence dans la plage 150-200 cpm) et à plus forte raison des FV (fréquence supérieure à 200 cpm), ces deux dernières situations nécessitant l'application sans délai d'une thérapie.

Le but de la présente invention est de remédier aux inconvénients précités, en proposant de perfectionner les appareils existant afin d'éliminer le risque de faux diagnostics de TS post-thérapie (TS interprétée à tort comme TV), de manière à accroître la spécificité et la fiabilité de l'analyse des tachyarythmies après application d'une thérapie.

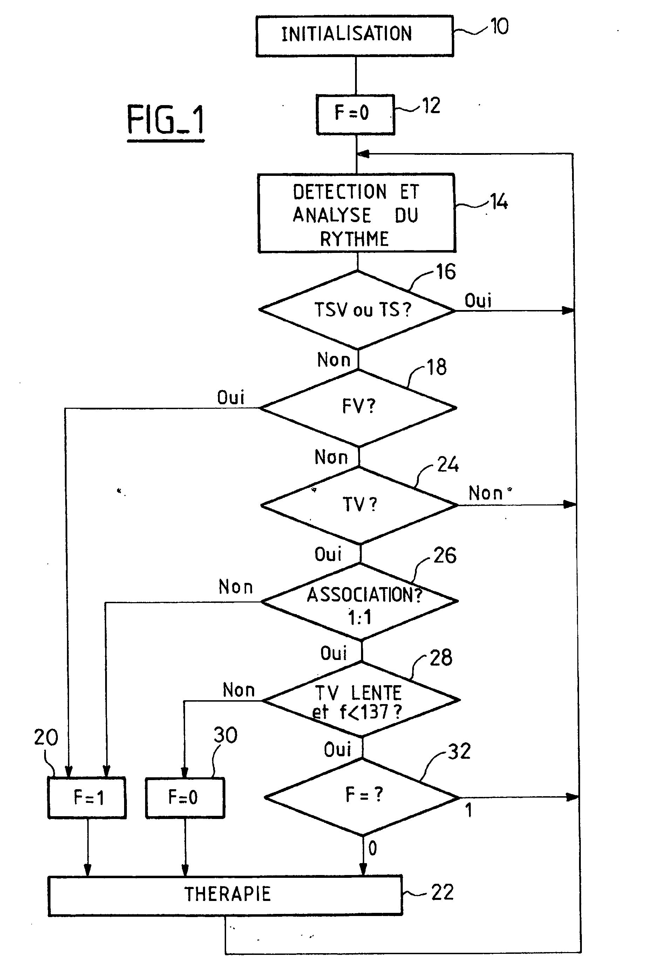

La figure 1 montre l'algorithme qui, selon l'invention, permet d'atteindre ce but.

Après initialisation des divers paramètres (étape 10), un indicateur F est positionné à '0' (étape 12).

L'étape suivante (étape 14) consiste à mettre en oeuvre l'algorithme de détection et de classification des tachyarythmies tel que décrit dans les EP-A-0 626 182 (algorithme de type PARAD) et EP-A-0 838 235 (algorithme de type PARAD+) précités.

A cette étape 14, l'algorithme est exécuté de la même manière que dans les dispositifs antérieurs.

Si le trouble est une TSV ou une TS (test de l'étape 16), comme dans l'art antérieur toute thérapie ventriculaire est inhibée, et ce de façon systématique. L'algorithme de détection et d'analyse 14 est ensuite réitéré (retour à l'étape 14). En cas de TSV, une thérapie auriculaire peut être envisagée.

Si le trouble est une FV (test de l'étape 18), comme dans l'art antérieur une thérapie est appliquée, ici encore de façon systématique, mais l'indicateur F est positionné à '1' (étape 20) avant l'application de cette thérapie (étape 22). L'algorithme de détection et d'analyse 14 est ensuite réitéré après application de la thérapie (retour à l'étape 14).

Dans l'hypothèse où le trouble n'est ni une TSV ni une TS ni une FV, on examine s'il s'agit d'une TV (test de l'étape 24) :
- dans la négative, la thérapie est inhibée et l'algorithme de détection et d'analyse est réitéré (retour à l'étape 14) ;
- dans l'affirmative, de façon caractéristique de l'invention le dispositif recherche s'il pourrait s'agir d'une TS post-thérapie, donc susceptible d'être interprétée (à tort) comme une TV.

Plus précisément, si l'on se trouve dans une configuration où l'on a :
1° association en 1:1 (test de l'étape 26) et
2° TV lente, par exemple une fréquence cardiaque inférieure à 137 cpm (test de l'étape 28), et
3° absence de thérapie appliquée à l'itération précédente (indicateur F positionné à F='0' (test de l'étape 32),
alors il s'agit d'une situation nécessitant l'application sans délai d'une thérapie, action réalisée à l'étape 22 et suivie d'un retour à l'étape 14 de détection et d'analyse à nouveau du rythme.

Si, à l'étape 26, l'analyse du rythme révélait une dissociation (conduction différente de 1:1), alors le dispositif positionne l'indicateur F à '1' (étape 20) et applique la thérapie (étape 22). Il convient de rappeler que la TV est diagnostiquée selon deux voies différentes : (i) RR stables/dissociation, ou (ii) RR stables/association 1:1/accélération du ventricule (cf. les enseignements du EP-A-0 626 182 précité).

Si, à l'étape 28, l'analyse du rythme révélait une TV rapide (typiquement une fréquence du rythme cardiaque f ≥ 137 cpm), alors le dispositif applique une thérapie (étape 22), mais en ayant au préalable positionné l'indicateur F à '0' (étape 30), c'est-à-dire que l'indicateur est maintenu à '0' s'il s'y trouvait, ou est rétabli à cette valeur s'il était auparavant positionné à '1'.

Si à l'étape 32, l'indicateur F était positionné à '1', indiquant qu'à l'itération précédente une thérapie avait été appliquée suite à une FV ou une TV avec dissociation, alors la thérapie est inhibée et l'algorithme de détection et d'analyse est réitéré (retour à l'étape 14). On notera en revanche que, dans le cas d'une TV rapide avec association 1:1 (étape 28), l'indicateur avait été positionné à '0' à l'étape 30 et que la thérapie ne sera pas inhibée car, dans ce cas particulier, elle doit être appliquée sans délai.

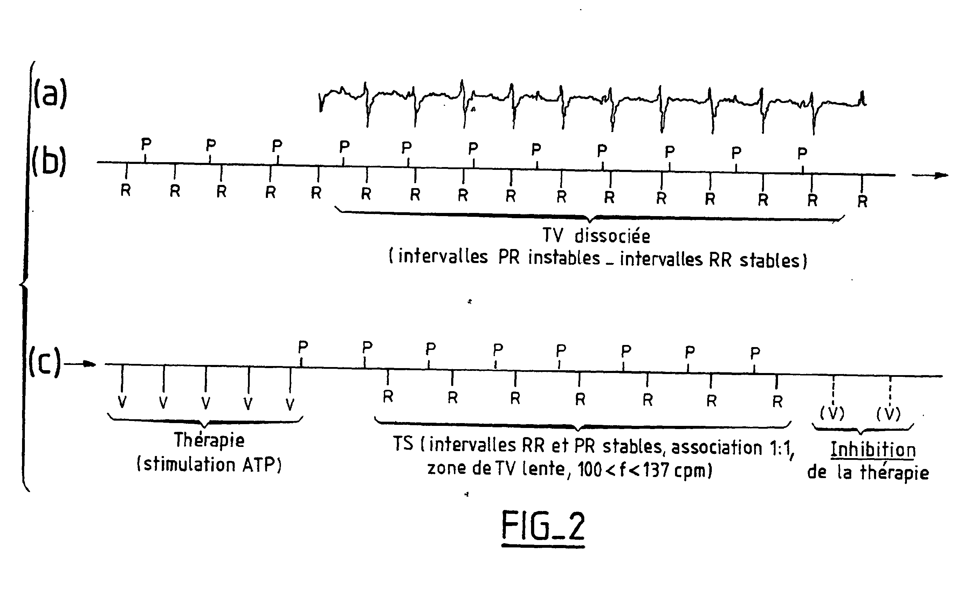

Les chronogrammes de la figure 2 illustrent le résultat de la mise en oeuvre de cet algorithme. La ligne (a) illustre une partie du tracé ECG, les lignes (b) et (c) - (c) étant la suite de (b) - indiquant les marqueurs d'événements auriculaires (détection auriculaire P) et ventriculaires (détection ventriculaire ou stimulation ventriculaire V).

La ligne (b) montre l'apparition d'une TV avec des intervalles RR stables mais des intervalles PR instables et absence de cycle long.

Cette situation va déclencher l'application d'une thérapie, ici sous forme de stimulation ATP correspondante à la série de stimuli ventriculaires V au début de la ligne (c).

Après application de cette thérapie, on a illustré l'apparition d'une TS post-thérapie, correspondant à une situation avec : intervalles RR et PR stables, association 1:1 et rythme cardiaque de 120 cpm (donc situé dans la zone de TV lente et inférieur à la fréquence limite préprogrammée de 137 cpm).

Dans une telle situation, l'algorithme de l'art antérieur aurait provoqué l'application d'une thérapie, illustré par les événements désignés (V) et illustrés en tiretés.

La modification apportée par l'algorithme de l'invention permet précisément, dans cette situation où une thérapie n'est pas nécessaire, d'inhiber l'application de celle-ci.

## Revendications

1. Un dispositif médical actif de type défibrillateur/cardioverteur implantable, comprenant :
- des moyens de recueil de l'activité ventriculaire et auriculaire,
- des moyens de détection d'épisodes de tachycardie dans l'activité ainsi recueillie,
- des moyens (22) de délivrance d'une thérapie de défibrillation et/ou de cardioversion et/ou de stimulation antitachycardique ventriculaire et/ou auriculaire,
- des moyens (14, 16, 18, 24, 26, 28, 30) de classification des tachycardies détectées par les moyens de détection, ces moyens de classification opérant de manière itérative et pouvant opérer la détection d'une tachycardie sinusale, une tachycardie supra-ventriculaire, ou une fibrillation ventriculaire, et
- des moyens inhibiteurs (16, 18, 24, 26, 28), aptes à inhiber sélectivement les moyens de délivrance de thérapie en fonction du type de tachycardie déterminé par les moyens de classification,
**caractérisé en ce que** :
- lesdits moyens (14, 16, 18, 24, 26, 28, 30) de classification des tachycardies comprennent des moyens de discrimination aptes, lors d'un itération faisant suite à l'application d'une thérapie, à déterminer la présence d'une tachycardie sinusale post-thérapie en cas de trouble classifié autre que tachycardie sinusale, tachycardie supra-ventriculaire ou fibrillation ventriculaire, et
- les moyens inhibiteurs (32) inhibent la délivrance d'une thérapie en cas de tachycardie sinusale post-thérapie déterminée par les moyens de discrimination.

2. Le dispositif de la revendication 1, dans lequel les moyens de classification des tachycardies determinent la présence de tachycardies sinusales post-thérapie par reconnaissance d'un rythme ventriculaire stable, présentant une association des rythmes ventriculaire et auriculaire en 1:1 et une fréquence cardiaque située dans une plage correspondant à une tachycardie ventriculaire lente comme déterminé par les moyens de classification.

3. Le dispositif de la revendication 2, dans lequel les moyens de classification des tachycardies déterminent la présence de tachycardies sinusales post-thérapie à condition que, en outre, la fréquence cardiaque (f) soit inférieure à une fréquence limite prédéterminée.

4. Le dispositif de la revendication 3, dans lequel il est prévu des moyens pour ajuster ladite fréquence limite prédéterminée à une valeur comprise entre 120 et 160 cpm.

5. Le dispositif de la revendication 1, dans lequel il est en outre prévu des moyens aptes, lorsque les moyens de classification déterminent une fibrillation ventriculaire (18), à :
- positionner (20) un indicateur (F) avant délivrance de la thérapie, et
- à l'itération suivante, en présence d'une tachycardie sinusale post-thérapie, inhiber les moyens de délivrance de thérapie si ledit indicateur est positionné, et délivrer la thérapie dans le cas contraire.

6. Le dispositif de la revendication 2, dans lequel il est en outre prévu des moyens aptes, lorsque les moyens de classification déterminent une tachycardie ventriculaire avec absence d'association (26), à :
- positionner (20) un indicateur (F) avant délivrance de la thérapie, et
- à l'itération suivante, en présence d'une tachycardie sinusale post-thérapie, inhiber les moyens de délivrance de thérapie si ledit indicateur (F) est positionné, et délivrer la thérapie dans le cas contraire.

7. Le dispositif de la revendication 5 ou 6, dans lequel il est en outre prévu des moyens aptes, lorsque les moyens de classification déterminent une fréquence cardiaque située hors de ladite plage correspondant à une tachycardie ventriculaire lente (28), à :
- s'il était positionné, dépositionner (30) ledit indicateur (F) avant délivrance de la thérapie, et
- à l'itération suivante, en présence d'une tachycardie sinusale post-thérapie, inhiber les moyens de délivrance de thérapie si ledit indicateur (F) est positionné, et délivrer la thérapie dans le cas contraire.

8. Le dispositif des revendications 3 et 5 prises en combinaison, ou des revendications 3 et 6 prises en combinaison, dans lequel il est en outre prévu des moyens (28, 30) pour, en cas de fréquence cardiaque (f) supérieure à la fréquence limite prédéterminée :
- s'il était positionné, dépositionner (30) ledit indicateur (F) avant délivrance de la thérapie, et
- à l'itération suivante, en présence d'une tachycardie sinusale post-thérapie, inhiber les moyens de délivrance de thérapie si ledit indicateur (F) est positionné, et délivrer la thérapie dans le cas contraire.

9. Le dispositif de la revendication 1, comprenant en outre des moyens de mémorisation permanente de la survenue d'une tachycardie sinusale post-thérapie déterminée par les moyens d'analyse.

## Claims

1. An implantable active medical device of the defibrillator/cardioverter type, comprising:
- means for collecting ventricular and atrial activity,
- means for detecting tachycardia episodes in the collected activity,
- means (22) for delivering a defibrillation and/or cardioversion therapy and/or a ventricular and/or atrial antitachycardia stimulation,
- means (14, 16, 18, 24, 26, 28, 30) for classifying the tachycardias detected by the detection means, said classification means operating in an iterative fashion and being able to perform the detection of a sinus tachycardia, a supraventricular tachycardia or a ventricular fibrillation, and
- inhibiting means (16, 18, 24, 26, 28), being able to selectively inhibit the therapy delivering means as a function of the tachycardia type determined by the classification means,
**characterised in that**:
said means (14, 16, 18, 24, 26, 28, 30) for classifying the tachycardias comprises discriminating means being able to determine, during an iteration following the application of a therapy, the presence of a post-therapy sinus tachycardia in the case of a disorder classified as different from a sinus tachycardia, a supraventricular tachycardia or a ventricular fibrillation, and
the inhibition means (32) inhibits the delivery of a therapy in the case of a post-therapy sinus tachycardia determined by the discriminating means.

2. The device of claim 1, wherein the means for classifying the tachycardias determines the presence of post-therapy sinus tachycardias by recognising a stable ventricular rhythm with a 1:1 association of the ventricular and atrial rhythms and a cardiac frequency situated in a range corresponding to a slow ventricular tachycardia as determined by the classification means.

3. The device of claim 2, wherein the means for classifying the tachycardias determines the presence of post-therapy sinus tachycardias provided that, furthermore, the cardiac frequency (f) is less than a predetermined threshold frequency.

4. The device of claim 3, wherein there is provided a means for adjusting said predetermined threshold frequency to a value of between 120 and 160 bpm.

5. The device of claim 1, wherein, furthermore, there is provided a means being able, when the classification means determines a ventricular fibrillation (18), to:
- position (20) an indicator (F) before the delivery of the therapy, and,
- at the next iteration, in the presence of a post-therapy sinus tachycardia, inhibit the means for delivering the therapy if the indicator is positioned, and deliver the therapy in the adverse case.

6. The device of claim 2, wherein, furthermore, there is provided a means being able, when the classification means determines a ventricular tachycardia with a lack of association (26), to:
- position (20) an indicator (F) before the delivery of the therapy, and,
- at the next iteration, in the presence of a post-therapy sinus tachycardia, inhibit the means for delivering the therapy if the indicator (F) is positioned, and deliver the therapy in the adverse case.

7. The device of claim 5 or 6, wherein, furthermore, there is provided a means being able, when the classification means determines a cardiac frequency located outside said range corresponding to a slow ventricular tachycardia (28), to:
- if it was positioned, deposition (30) said indicator (F) before delivery of the therapy, and,
- at the next iteration, in the presence of a post-therapy sinus tachycardia, inhibit the means for delivering the therapy if the indicator (F) is positioned, and deliver the therapy in the adverse case.

8. The device of claims 3 and 5 in combination or of claims 3 and 6 in combination, wherein, furthermore, there is provided a means (28, 30) in order to, if the cardiac frequency (f) is higher than the predetermined threshold frequency:
- if it was positioned, deposition (30) said indicator (F) before delivery of the therapy, and,
- at the next iteration, in the presence of a post-therapy sinus tachycardia, inhibit the means for delivering the therapy if the indicator (F) is positioned, and deliver the therapy in the adverse case.

9. The device of claim 1, further comprising means for permanently memorising the advent of a post-therapy sinus tachycardia determined by the classification means.

## Patentansprüche

1. Aktive medizinische Vorrichtung vom implantierbaren Defibrillator/Kardioverter-Typ, mit:
― Mitteln zur Aufnahme der Herzkammer- und Herzvorhofaktivität,
― Mitteln zur Erfassung von Tachykardie-Episoden in der so aufgenommenen Aktivität,
― Mitteln (22) zur Abgabe einer Defibrillations- und/oder Kardioversionstherapie und/oder zur antitachykardischen Herzkammer- und/oder Herivorhofstimulation,
― Mitteln (14,16, 18, 24, 26, 28, 30) zur Klassifikation der durch die Er-<SHY>(14,16, 18, 24, 26, 28, 30) zur Klassifikation der durch die Erfassungsmittel erfassten Tachykardien, wobei diese Klassifikationsmittel iterativ arbeiten und die Erfassung einer Sinustachykardie, einer supraventrikulären Tachykardie oder eines Kammerflimmern durchführen können, und
― Inhibitionsmitteln (16, 18, 24, 26, 28), die geeignet sind, selektiv die Mittel zur Therapieabgabe in Abhängigkeit des durch die Klassifikationsmittel festgestellten Tachykardie-Typs zu inhibieren,
**dadurch gekennzeichnet, dass**:
- die Mittel (14, 16, 18, 24, 26, 28, 30) zur Klassifikation der Tachykardien Unterschcidungsmittel umfassen, die geeignet sind, während einer auf die Anwendung einer Therapie folgenden Iteration das Vorliegen einer post-therapeutischen Sinustachykardie im Falle einer Störung festzustcllen, die anders als eine Sinustachykardie, eine supraventrikuläre Tachykardie oder ein Kammerflümmem klassifiziert ist, und
- die Inhibitionsmittel (32) das Abgeben einer Therapie im Falle einer durch die Unterscheidungsmittel festgestellten post-therapeutischen Sinustachykardie inhibieren.

2. Vorrichtung nach Anspruch 1, bei welcher die Mittel zur Klassifikation der Tachykardien das Vorliegen von post-therapeutischen Sinustachykardien durch Erkennung eines stabilen Herzkammerrhythmus feststellen, der eine 1:1-Verbindung der Herzkammer- und Herzvorhofrhythmen und eine Herzfrequenz aufweist, die in einem einer langsamen ventrikulären Tachykardie entsprechenden Bereich liegt, wie es durch die Klassifikationsmittel festgestellt wird.

3. Vorrichtung nach Anspruch 2, bei welcher die Mittel zur Klassifikation der Tachykardien das Vorliegen von post-therapeutischen Sinustachykardien unter der Bedingung feststellen, dass außerdem die Herzfrequenz (f) geringer als eine vorbestimmte Grenzfrequenz ist.

4. Vorrichtung nach Anspruch 3, bei welcher Mittel vorgesehen sind, um die vorbestimmte Grenzfrequenz auf einen Wert zwischen 120 und 160 cpm einzustellen.

5. Vorrichtung nach Anspruch 1, bei welcher weiterhin Mittel vorgesehen sind, die, wenn die Klassifikationsmittel ein Kammerflimmern feststellen (18), zu folgendem geeignet sind:
- Positionieren (20) eines Indikators (F) vor Abgabe der Therapie, und
- bei der nächsten Iteration, bei Vorliegen einer post-therapeutischen Sinustachykardie, Inhibieren der Mittel zur Therapieabgabe, wenn der Indikator positioniert ist, und Abgabe der Therapie im gegenteiligen Fall.

6. Vorrichtung nach Anspruch 2, bei welcher weiterhin Mittel vorgesehen sind, die, wenn die Klassifikationsmittel eine ventrikulärc Tachykardie unter Abwesenheit einer Verbindung feststellen (26), zu folgendem geeignet sind:
- Positionieren (20) eines Indikators (F) vor Abgabe der Therapie, und
- bei der nächsten Iteration, bei Vorliegen einer post-therapeutischen Sinustachykardie, Inhibieren der Mittel zur Therapieabgabe, wenn der Indikator (F) positioniert ist, und Abgabe der Therapie im gegenteiligen Fall.

7. Vorrichtung nach Anspruch 5 oder 6, bei welcher weiterhin Mittel vorgesehen sind, die, wenn die Klassifikationsmittel eine Herzfrequenz feststellen, die außerhalb des einer langsamen ventrikulären Tachykardie entsprechenden Bereichs liegt (28), zu folgendem geeignet sind:
- wenn er positioniert war, Entpositionieren (30) des Indikators (F) vor Abgabe der Therapie, und
- bei der nächsten Iteration, bei Vorliegen einer post-therapeutischen Sinustachykardie, Inhibieren der Mittel zur Therapieabgabe, wenn der Indikator (F) positioniert ist, und Abgabe der Therapie im gegenteiligen Fall.

8. Vorrichtung nach den Ansprüchen 3 und 5 in Kombination oder den Ansprüchen 3 und 6 in Kombination, bei welcher weiterhin Mittel (28, 30) vorgesehen sind, um im Falle einer über der vorbestimmten Grenzfrequenz liegenden Herzfrequenz (f):
- wenn er positioniert war, den Indikator (F) vor Abgabe der Therapie zu entpositionieren (30), und
- bei der nächsten Iteration, bei Vorliegen einer post-ttxerapeutischen Sinustachykardie, die Mittel zur Therapicabgabe zu inhibieren, wenn der Indikator (F) positioniert ist, und die Therapie im gegenteiligen Fall abzugeben.

9. Vorrichtung nach Anspruch 1, weiterhin mit Mitteln zur dauerhaften Speicherung des Auftretens einer durch die Klassifikationsmittel festgestellten post-therapeutischen Sinustachykardie.
